Europäisches Patentamt

European Patent Office

Office européen des brevets

(11) Veröffentlichungsnummer: **0 285 152 B1**

(12) **EUROPÄISCHE PATENTSCHRIFT**

(45) Veröffentlichungstag der Patentschrift: **07.01.93**

(21) Anmeldenummer: **88105219.5**

(22) Anmeldetag: **30.03.88**

(51) Int. Cl.⁵: **C12N 15/70**

(54) **Rekombinante DNA zur reprimierbaren und induzierbaren Expression von Fremdgenen.**

(30) Priorität: **31.03.87 DE 3710633**
**19.10.87 DE 3735381**

(43) Veröffentlichungstag der Anmeldung:
**05.10.88 Patentblatt 88/40**

(45) Bekanntmachung des Hinweises auf die
Patenterteilung:
**07.01.93 Patentblatt 93/01**

(84) Benannte Vertragsstaaten:
**AT BE CH DE ES FR GB GR IT LI LU NL SE**

(56) Entgegenhaltungen:

**ARCH. MICROBIOL., Band 148, Nr. 1, 1987, Seiten 44-51, Springer-Verlag; A. BIRKMANN et al.: "Factors affecting transcriptional regulation of the formate-hydrogen-lyase pathway of Escherichia coli"**

**MOL. GEN. GENOT, Band 210, 1987, Seiten 535-542, Springer-Verlag; A. BIRKMANN et al.: "Involvement of the ntrA gene product in the anaerobic metabolism of Escherichia coli"**

(73) Patentinhaber: **BOEHRINGER MANNHEIM GMBH**
**Patentabteilung, Abt. E Sandhofer Strasse 112-132 Postfach 31 01 20**
**W-6800 Mannheim 31 Waldhof(DE)**

(72) Erfinder: **Birkmann, Angelika**
**Herzogstandstrasse 6**
**W-8000 München(DE)**
Erfinder: **Böck, August, Prof. Dr.**
**Lindenstrasse 10**
**W-8085 Geltendorf 2(DE)**

(74) Vertreter: **Huber, Bernhard, Dipl.-Chem. et al**
**Patentanwälte H. Weickmann, Dr. K. Fincke F.A. Weickmann, B. Huber Dr. H. Liska, Dr. J. Prechtel Kopernikusstrasse 9 Postfach 86 08 20**
**W-8000 München 86(DE)**

CELL, Band 45, 1986, Seiten 785-792; L.J. REITZER et al.: "Transcription of ginA in E. coli is stimulated by activator bound to sites far from the promoter"

PROC. NATL. ACAD. SCI., Band 82, November 1985, Seiten 7525-7529, US; J. HIRSCHMAN et al.: "Products of nitrogen regulatory genes ntrA and ntrC of enteric bacteria activate glnA transcription in vitro: evidence that the ntrA product is a o factor"

NUCLEIC ACIDS RESEARCH, Band 13, Nr. 21, 1985, Seiten 7621-7636; M. BUCK et al.: "Site-directed mutagenesis of the Klebsiella pneumoniae nifL and nifH promoters and in vivo analysis of promoter activity"

ANN. REV. GENET., Band 20, 1986, Seiten 567-591, Annual Reviews Inc.; G.N. GUSSIN et al.: "Regulationn of nitrogen fixation genes"

**Beschreibung**

Die Erfindung betrifft rekombinante DNA und Expressionsvektoren, Verfahren zur Herstellung solcher rekombinanter DNA und Expressionsvektoren sowie deren Verwendung zur induzierbaren und reprimierbaren Expression eines Fremdgens.

Ein wichtiges Ziel der angewandten Gentechnologie ist die Proteinproduktion aus rekombinanter DNA. Dazu wird eine besondere Klasse von Vektoren, die sogenannten Expressionsvektoren, benötigt. Diese besitzen nicht nur die strukturellen Voraussetzungen für die Klonierung, den Transfer und die Vermehrung der rekombinanten DNA, sondern auch für die Expression in ein Protein. Hierzu benötigen diese rekombinanten DNA-Moleküle spezielle Regulationssequenzen, sogenannte Promotoren, welche die Transkription der DNA-Sequenz in RNA bewirken, deren Translation durch die Ribosomen zum fertigen Protein führt.

Als Promotoren werden DNA-Regionen bezeichnet, an die bakterielle RNA-Polymerase zur Transkription eines oder mehrerer Gene binden muß. Viele solche Promotoren haben strukturelle Gemeinsamkeiten, deren Bedeutung u. a. in der Interaktion mit bestimmten Proteinen vermutet wird. Durch solche Interaktionen mit zellulären Proteinen oder anderen Molekülen kann eine Repression, jedoch auch eine Induktion der Aktivität eines Promotors bewirkt werden. Ein Beispiel dafür ist beispielsweise die Interaktion des Lambda-Promotors $P_1$ mit dem Lambda-Repressor cl.

Bei der gentechnologischen Produktion von Proteinen ist es von besonderem Vorteil, wenn ein in einem Expressionsvektor vorhandener Promotor durch Vorhandensein oder Zugabe von Repressor oder Induktor reguliert werden kann. Bisher ist es jedoch nur durch Zugabe von Induktor oder durch Verwendung von temperatursensitiven Mikroorganismen und einer genau eingehaltenen Temperaturveränderung möglich, eine solche Repression oder Induktion zu bewirken. Die verwendeten Induktoren sind meist sehr teuer und die angewandten Verfahren zur Induktion oder Repression kompliziert und nur bedingt zur Regulation der Expression eines Proteins geeignet. Es ist daher Aufgabe dieser Erfindung, rekombinante DNA und Expressionsvektoren bereitzustellen, die auf möglichst einfache Art und Weise eine Regulation der Expression eines gewünschten Genprodukts sowie eine erhöhte Expressionsrate des Genprodukts ermöglichen.

Gelöst wird diese Aufgabe durch rekombinante DNA, die dadurch gekennzeichnet ist, daß sie die Consensus-Sequenz

```
     T   TTTC    T         A A   T
 AT   TCG      G   CACGTC A AC G
     G   AAAT    A         G C   G
```

und einen Promoter, der die DNA-Sequenz $GGN_{10}GC$ aufweist, enthält.

Durch das Vorhandensein dieser DNA-Consensus-Sequenz wird bewirkt, daß ein dahinterliegender Promotor, der die DNA-Sequenz $GGN_{10}GC$ aufweist, durch Sauerstoff reprimiert, dagegen durch Formiat unter anaeroben Bedingungen induziert wird.

Bevorzugt befindet sich diese DNA-Consensus-Sequenz 15 bis 150 Basenpaare upstream (d. h.auf der 5′-Seite) vom Transkriptionsstart eines unter der Kontrolle des Promotors exprimierten Gens, wobei sich der Promotor zwischen der Consensus-Sequenz und dem Transkriptionsstart befindet. Besonders bevorzugt liegt die Consensus-Sequenz 80 bis 130 Basenpaare vor dem Tranksriptionsstart.

Als Promotoren geeignet sind der fdhF-Promotor, ein Promoter einer für die Hydrogenaseexpression essentiellen Transktionseinheit oder essentielle Strukturelemente der ntr-A-abhängigen Promotoren, soweit sie die obengenannte DNA-Sequenz enthalten. Bevorzugt wird der fdhF-Promotor verwendet.

Ein weiterer Gegenstand der Erfindung ist ein Expressionsvektor, der die erfindungsgemäße rekombinante DNA einligiert in einen geeigneten Vektor enthält. Als Vektoren können hierbei üblicherweise verwendete Vektoren wie z. B. pBR322 oder pUC- Vektoren verwendet werden. Zusätzlich kann ein erfindungsgemäßer Expressionsvektor hinter dem Promotor einen Polylinker enthalten, der das Einsetzen eines beliebigen Fremdgens durch das Vorhandensein von mehreren Restriktionsschnittstellen erleichtert.

Ein weiterer Gegenstand der Erfindung ist das Plasmid pBN80, DSM 4073P, das dadurch gekennzeichnet ist, daß es den upstream-Bereich des fdhF-Gens ab -240 Basenpaare vor dem Translationsstart, enthält. In diesem upstream-Bereich des fdhF-Gens ist sowohl die obengenannte DNA-Consensus-Sequenz, sowie der fdhF-Promotor, der die DNA-Sequenz $GGN_{10}GC$ aufweist, enthalten. Dieses Plasmid zeigt durch $O_2$ reprimierbare und durch Formiat unter anaeroben Bedingungen induzierbare Tetrazyklinresistenz. Es ist jedoch auch weiterhin möglich, durch Spaltung mit geeigneten Restriktionsnukleasen und Ligieren ein weiteres Fremdgen in dieses Plasmid zu insertieren und dessen Expression auf die obengenannte Weise zu steuern.

EP 0 285 152 B1

Ein weiterer Gegenstand der Erfindung ist ein Verfahren zur Herstellung der erfindungsgemäßen rekombinanten DNA, das dadurch gekennzeichnet ist, daß man eine die Consensus-Sequenz enthaltende DNA-Sequenz aus einer Genbank eines Mikroorganismus, der ein Gen enthält, das durch $O_2$ reprimierbar und unter anaeroben Bedingungen durch Formiat induzierbar ist, nach bekannten Methoden identifiziert, isoliert und mit einem geeigneten Promotor verbindet.

Zur Identifikation dieser DNA-Sequenz kann z. B. eine Hybridisierung oder Kopplung an ein Indikatorgen und Überprüfen der Expression unter aeroben und anaeroben Bedingungen, durchgeführt werden.

Bevorzugt wird die DNA-Sequenz aus E. coli, DSM 2093, DSM 2102 oder Salmonella typhimurium, DSM 554, isoliert. Analog zu diesem Verfahren zur Herstellung einer rekombinanten DNA kann man auch anstelle eines DNA-Fragments, welches nur die Consensus-Sequenz enthält, die gesamte upstream-Region eines durch Formiat induzierbaren und durch $O_2$ reprimierbaren Gens, das sowohl die Consensus-Sequenz als auch einen Promotor enthält, der wiederum die DNA-Sequenz $GGN_{10}GC$ enthält, isolieren. Bevorzugt geschieht dies, indem man die upstream-Region des fdhF-Gens oder einer für die Hydrogenaseexpression essentiellen Transkriptionseinheit isoliert.

Ein weiterer Gegenstand der Erfindung ist ein Verfahren zur Herstellung eines erfindungsgemäßen Expressionsvektors, der die erfindungsgemäße rekombinante DNA enthält, wobei man die wie oben isolierte rekombinante DNA-Sequenz, die die Consensus-Sequenz, verbunden mit einem geeigneten Promotor, oder die upstream-Region eines durch Formiat induzierbaren und durch $O_2$ reprimierbaren Gens, welche sowohl die Consensus-Sequenz als auch einen Promotor enthält, gegebenenfalls zusätzlich mit einem Polylinker in einen geeigneten Vektor insertiert. Als Vektoren kommen hierbei die obengenannten Vektoren, aber auch alle übrigen zur Expression von Fremdgenen geeigneten Vektoren in Frage.

Das erfindungsgemäße Verfahren zur Herstellung des Plasmids pBN80, DSM 4073P, ist dadurch gekennzeichnet, daß man die upstream-Region des fdhF-Gens ab -240 bp vor dem Translationsstart in den mit PstI und BglII geschnittenen Vektor pGA46, DSM 4068P, insertiert.

Die erfindungsgemäße Verwendung einer rekombinanten DNA oder eines Expressionsvektors, wie sie oben beschrieben sind, zur induzierbaren und reprimierbaren Expression eines Fremdgens, ist dadurch gekennzeichnet, daß die Induktion unter anaeroben Bedingungen durch Formiat, die Repression durch $O_2$ bewirkt wird.

Die Expression kann hierbei in einem dafür geeigneten Mikroorganismus der Gattung Enterobacteriaceae, bevorzugt in E. coli oder Salmonella typhimurium durchgeführt werden.

Bevorzugt wird die Expression in einem Wirtsstamm durchgeführt, der ntrA positiv ist.

Das ntrA-Genprodukt spielt im anaeroben Metabolismus von Mikroorganismen eine wichtige Rolle. Das ntrA-Genprodukt ist nämlich ein Sigma-Faktor, der die Promotorselektivität des RNA-Polymeraseenzyms derart verändert, daß es die in der rekombinanten DNA und Expressionsvektoren der Stammanmeldung enthaltenen Consensus- und Promotorsequenzen besser erkennen kann. Die erfindungsgemäße Verwendung der rekombinanten DNA oder Expressionsvektoren der Stammanmeldung ist zwar auch ohne das Vorhandensein des ntrA-Genprodukts möglich, das ntrA-Genprodukt verstärkt jedoch die Expression des Fremdgens ganz wesentlich.

Wird ein Wirtsstamm verwendet, der ntrA⁻ ist, gibt es zwei Möglichkeiten, der Zelle trotzdem das ntrA-Genprodukt zuzufühen. Eine Möglichkeit ist, das ntrA-Gen in die rekombinante DNA oder den Expressionsvektor, der auch das gewünschte Fremdgen trägt, einzuligieren, und somit gleichzeitig mit der Expression des Fremdgens auch die Expression des ntrA-Genproduktes zu erreichen, oder aber das ntrA-Gen auf einem zusätzlichen Vektor in die Wirtszellen einzubringen. Die Produktion des ntrA-Gens ist dann unabhängig von der des Fremdgens, diese wird jedoch durch das ntrA-Genprodukt positiv beeinflußt.

Besonders bevorzugt wird zum Einbringen in eine Wirtszelle über entweder die rekombinante DNA oder den Expressionsvektor oder aber auf einem zusätzlichen Vektor ein dem der Wirtszelle entsprechendes ntrA-Gen verwendet. Hiermit werden bei der Expression des Fremdgens bedeutend bessere Ergebnisse erzielt als bei Verwendung eines artfremden ntrA-Gens (Tabelle I, letzte Zeile).

Durch erfindungsgemäße rekombinante DNA und Expressionsvektoren ist es auf einfache Art und Weise möglich, die Expression eines Fremdgens zu regulieren. So wird beispielsweise in der aeroben frühen Wachstumsphase des verwendeten Mikroorganismus eine möglicherweise störende Expression des Fremdgens unterdrückt. Beim Übergang in die späte logarithmische, anaerobe Wachstumsphase wird die Expression dann durch vom Mikroorganismus gebildetes Formiat induziert und durch Zugabe von Formiat ins Wachstumsmedium der Mikroorganismen eine Verstärkung der Expression erreicht. Auf diese Art und Weise kann vermieden werden, daß Zugabe von Induktor Zellen in unterschiedlichen Stadien der Wachstumsphase zur Expression des Fremdgens anregt, was ein weiteres Wachstum eventuell verhindern kann und auf die Zelle selbst negative Auswirkungen haben könnte. In der anaeroben späten logarithmischen Wachstumsphase sind die Zellen bereits hochgewachsen und haben eine optimale Dichte erreicht. Dabei

4

tritt automatisch eine Sauerstofflimitierung ein, die fermentationstechnisch noch verstärkt oder reguliert werden kann. Durch die hohe Zelldichte der Mikroorganismen kann eine optimale Expression des Fremdgens erreicht werden.

Die Verwendung der erfindungsgemäßen Expressionsvektoren zur Produktion von Proteinen von hohem Interesse stellt daher eine kostengünstige und einfache Möglichkeit zur Regulation dar, da teure und komplizierte Induktion (Induktorzugabe, Temperaturshift usw.) nicht mehr notwendig ist.

Die folgenden Beispiele in Verbindung mit den Figuren erläutern die Erfindung weiter.

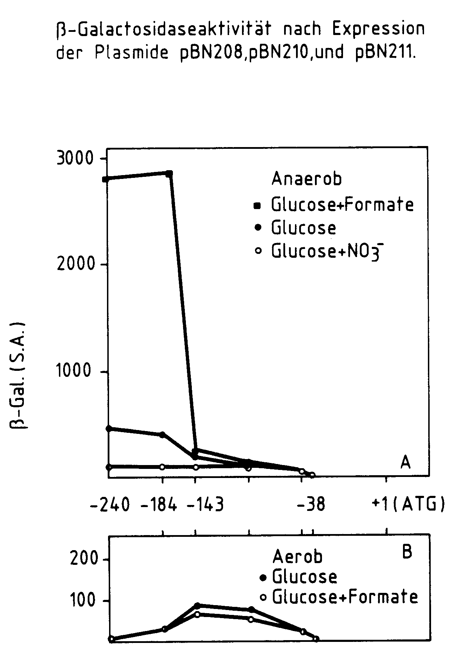

Fig. 1    zeigt die $\beta$-Galactosidaseaktivität nach Expression der Plasmide pBN 208, pBN 210 und pBN 211 unter anaeroben (A) und unter aeroben (B) Wachstumsbedingungen.

**Beispiel 1**

Konstruktion von fdhF-lac-Z-Fusionsplasmiden.

Das AatII-Fragment (ungefähr 1,5 kb) des Plasmids pBN2, DSM 4072P, einem fdhF- lacZ-Fusionsplasmid, (Proc. Natl. Acad, Sci. USA 83, (1986) 4650-4654) wurde durch präparative Agarose-Gelelektrophorese isoliert und unterschiedlich lange mit 0,2 U/μg DNA Bal31 Exonuclease bei 20°C inkubiert. Nach dem Auffüllen der überhängenden 5′-Enden mit DNA-Polymerase (Klenow-Fragment) wurden die Fragmente mit EcoRI-Linkern (dGGAATTCC) ligiert und nachfolgend mit den Restriktionsendonucleasen EcoRI und BamHI verdaut. Durch präparative Agarose-Gelelektrophorese wurde das Fragmentgemisch aufgetrennt und drei Größenfraktionen (Vergleich mit Restriktionsfragmenten bekannter Größe als Längenmarker) wurden aus dem Gel eluiert. Diese Fragmente wurden in die Multilinkerstelle des Plasmids pMC1403, DSM 4067P, kloniert. Als Rezipient wurde hierbei E.coli FM 911, DSM 4066P (MC 4100, fdhF, rec A56, lac⁻), verwendet. Hieraus ergaben sich Klone, die von der 5′-Seite der upstream-Region her verkürzt waren. In einem zweiten Schritt wurde der kürzeste Klon, pBN208, DSM 4069P, isoliert, durch Verdauung mit EcoRI linearisiert und mit 0,1 U/μg DNA Bal31 bei 20°C inkubiert. Nach Auffüllen der überhängenden 5′-Enden wurden wieder EcoRI-Linker anligiert und in der Folge wurde die DNA mit EcoRI und ClaI geschnitten. Es ergab sich eine Serie von Fragmenten, die in den EcoRI/ClaI-verdauten Vektor pMC1403, DSM 4067P, zurückkloniert wurden. Die erhaltenen Plasmide wurden erneut in den E.coli-Stamm FM 911, transformiert. Die beiden Klone, pBN210, DSM 4070P, und pBN211, DSM 4071P, wurden isoliert.

Die Transformation der Plasmide pBN2, pBN208, pBN210, pBN211, pMC1403 in den Stamm FM911 (MC4100, fdhF, rec A56) wurde im wesentlichen nach der von Cohen et al. beschriebenen Methode (Cohen et al. (1977), PNAS 69: 2110-2114) durchgeführt:

Früh logarithmische Zellen werden nach Abkühlung auf 0°C geerntet und anschließend einer Behandlung mit eiskaltem CaCl₂ (50mM) unterzogen. Die Zugabe der DNS erfolgt bei 0°C und nach einer Inkubation von 45 Minuten bei 0°C wird ein Hitzeschock (4 Minuten, 43°C) durchgeführt. Nach Abkühlen auf Raumtemperatur werden die Zellen in Vollmedium bei 37°C 1 Stunde zur phänotypischen Expression inkubiert. Die Selektion nach Ampicillinresistenz bei den Plasmiden pBN2, pBN208, pBN210, pBN211, pMC1403, erfolgt auf Platten mit 100μg/ml Ampicillin.

Die genauen Verbindungspunkte von Vektor mit dem fdhF-DNA-Fragment, d. h. die Position der EcoRI-Linker wurde durch DNA-Sequenzierung nach der Methode von Maxam und Gilbert, Methods of Enzymology 65, 499-560 (1980) durchgeführt.

Die Nucleotidsequenz des fdhF-Gens, einschließlich des 5′ upstream-Bereichs wird im folgenden gezeigt. ATG bezeichnet den Start der Translation. Aus der Figur kann man die verbleibende Sequenz des fdhF-Bereichs für die Plasmide pBN208, pBN210 und pBN211 entnehmen.

Sequenz von fdhF

```
(SmaI)
        10        20        30        40        50        60        70        80
GGGCGCTGCCGGCACCTGTCCTACGAGTTGCATGATAAAGAAGACAGTCATAAGTGCGGCGACGATAGTCATGCCCCGCG

        90       100       110       120       130       140       150       160
CCCACCGGAAGGAGCTACCGGCAGCGGTGCGGACTGTTGTAACTCAGAATAAGAAATGAGGCCGCTCATGGCGTTGGTCT

       170       180       190       200       210       220       230       240
GAAATTGCCGCTGTTTGACGGTGGACGGTTGAATGCCAATCTCGAAGGCACGCGCGCCGCCAGCAACATGATGATTGAAC

       250       260       270       280       290       300       310       320
GTTACAACCAGTCAGTACTGAACGCGGTGCGTGACGTTGCCGTCAACGGCACGCGTCTGCAAACGCTCAACGACGAGCGA

       330       340       350       360       370       380       390       400
GAAATGCAGGCTGAACGCGTGGAAGCCACGCGCTTTACCCAGCGCGCTGCCGAGGCCGCCTATCAGCGCGGCTTAACCAG

       410       420       430       440       450       460       470       480
CCGCTTACAGGCCACCGAAGCCCGGTTGCCAGTGCTTGCCGAAGAGATGTCATTACTGATGCTGGACAGCCGCCGGGTGA
                                    ┌──── pBN208
       490       500       510       520       530       540       550       560
TCCAAAGCATTCAGTTGATGAAATCGCTGGGCGGCGGGTATCAGGCAGGTCCCGTCGTCGAGAAAAAATAAAATGTCTGC
       ┌──── pBN210                              ┌──── pBN211
       570       580       590       600       610       620       630       640
CGCGTGATGGCTGTCACGCGGTATTTCGTTTCGTCACGTCAAAACTGACGACAGCCTGTTTTTCGTCAGAGTTTTGAATA
                                               ┌────→ mRNA
       650       660       670       680       690       700       710       720
AATAGTGCCCGTAATATCAGGGAATGACCCCACATAAAATGTGGCATAAAAGATGCATACTGTAGTCGAGAGCGCGTATG

       730       740       750       760       770       780       790       800
CGTGATTTGATTAACTGGAGCGAGACCGATGAAAAAAGTCGTCACGGTTTGCCCCTATTGCGCATCAGGTTGCAAAATCA
               SD         ATG                                  KpnI
       810       820       830       840       850       860       870       880
ACCTGGTCGTCGATAACGGCAAAATCGTCCGGGCGGAGGCAGCGCAGGGGAAAAACCAACCAGGGTACCCTGTGTCTGAAG
                                                                  pBN2
       890       900       910       920       930       940       950       960
GGTTATTATGGCTGGGACTTCATTAACGATACCCAGATCCTGACCCCGCGCCTGAAAACCCCCATGATCCGTCGCCAGCG

       970       980       990      1000      1010      1020      1030      1040
TGGCGGCAAACTCGAACCTGTTTCCTGGGATGAGGCACTGAATTACGTTGCCGAGCGCCTGAGCGCCATCAAAGAGAAGT

      1050      1060      1070      1080      1090      1100      1110      1120
ACGGTCCGGATGCCATCCAGACGACCGGCTCCTCGCGTGGTACGGGTAACGAAACCAACTATGTAATGCAAAAATTTGCG

      1130      1140      1150      1160      1170      1180      1190      1200
CGCGCCGTTATTGGTACCAATAACGTTGACTGCTGCGCTCGTGTCTGACACGGCCCATCGGTTGCAGGTCTGCACCAATC

      1210      1220      1230      1240      1250      1260      1270      1280
GGTCGGTAATGGCGCAATGAGCAATGCTATTAACGAAATTGATAATACCGATTTAGTGTTCGTTTTCGGGTACAACCCGG

      1290      1300      1310      1320      1330      1340      1350      1360
CGGATTCCCACCCAATCGTGGCGAATCACGTAATTAACGCTAAACGTAACGGGGCGAAAATTATCGTCTGCGATCCGCGC

      1370      1380      1390      1400      1410      1420      1430      1440
AAAATTGAAACCGCGCGCATTGCTGACATGCACATTGCACTGAAAAACGGCTCGAACATCGCGCTGTTGAATGCGATGGG

      1450      1460      1470      1480      1490      1500      1510      1520
CCATGTCATTATTGAAGAAAATCTGTACGACAAAGCGTTCGTCGCTTCACGTACAGAAGGCTTTGAAGAGTATCGTAAAA
```

6

```
         1530      1540      1550      1560      1570      1580      1590      1600
TCGTTGAAGGCTACACGCCGGAGTCGGTTGAAGATATCACCGGCGTCAGCGCCAGTGAGATTCGTCAGGCGGCACGGATG

         1610      1620      1630      1640      1650      1660      1670      1680
TATGCCCAGGCGAAAAGCGCCGCCATCCTGTGGGGCATGGGTGTAACCCAGTTCTACCAGGGCGTGGAAACCGTGCGTTC

         1690      1700      1710      1720      1730      1740      1750      1760
TCTGACCAGCCTCGCGATGCTGACCGGTAACCTCGGTAAGCCGCATGCGGGTGTTAACCCGGTTCGTGGTCAGAACAACG

         1770      1780      1790      1800      1810      1820      1830      1840
TTCAGGGTGCCTGCGATATGGGCGCGCTGCCGGATACGTATCCGGGATACCAGTACGTGAAAGATCCGGCTAACCGCGAG

         1850      1860      1870      1880      1890      1900      1910      1920
AAATTCGCCAAAGCCTGGGGCGTGGAAAGCCTGCCAGCGCATACCGGCTATCGCATCAGCGAGCTGCCGCACCGCGCAGC

         1930      1940      1950      1960      1970      1980      1990      2000
GCATGGCGAAGTGCGTGCCGCGTACATTATGGGCGAAGATCCGCTACAAACTGACGCGGAGCTGTCGGCAGTACGTAAAG

         2010      2020      2030      2040      2050      2060      2070      2080
CCTTTGAAGATCTGGAACTGGTTATCGTTCAGGACATCTTTATGACCAAAACCGCGTCGGCGGCGGATGTTATTTTACCG

         2090      2100      2110      2120      2130      2140      2150      2160
TCAACGTCGTGGGGCGAGCATGAAGGCGTGTTTACTGCGGCTGACCGTGGCTTCCAGCGTTTCTTCAAGGCGGTTGAACC

         2170      2180      2190      2200      2210      2220      2230      2240
GAAATGGGATCTGAAAACGGACTGGCAAATCATCAGTGAAATCGCCACCCGTATGGGTTATCCGATGCACTACAACAACA

         2250      2260      2270      2280      2290      2300      2310      2320  .
CCCAGGAGATCTGGGATGAGTTGCGTCATCTGTGCCCGGATTTCTACGGTGCGACTTACGAGAAAATGGGCGAACTGGGC

         2330      2340      2350      2360     ·2370      2380      2390      2400
TTCATTCAGTGGCCTTGCCGCGATACTTCAGATGCCGATCAGGGGACTTCTTATCTGTTTAAAGAGAAGTTTGATACCCC

         2410      2420      2430      2440      2450      2460      2470      2480
GAACGGTCTGGCGCAGTTCTTCACCTGCGACTGGGTAGCGCCAATCGACAAACTCACCGACGAGTACCCGATGGTACTGT

         2490      2500      2510      2520      2530      2540      2550      2560
CAACGGTGCGTGAAGTTGGTCACTACTCTTGCCGTTCGATGACCGGTAACTGTGCGGCACTGGCGGCGCTGGCTGATGAA

         2570      2580      2590      2600      2610      2620      2630      2640
CCTGGCTACGCACAAATCAATACCGAAGACGCCAAACGTCTGGGTATTGAAGATGAGGCATTGGTTTGGGTGCACTCGCG

         2650      2660      2670      2680      2690      2700      2710      2720
TAAAGGCAAAATTATCACCCGTGCGCAGGTCAGCGATCGTCCGAACAAAGGGGCGATTTACATGACCTACCAGTGGTGGA

         2730      2740      2750      2760      2770      2780      2790      2800
TTGGTGCCTGTAACGAGCTGGTTACCGAAAACTTAAGCCCGATTACGAAAACGCCGGAGTACAAATACTGCGCCGTTCGC

         2810      2820      2830      2840      2850      2860      2870      2880
GTCGAGCCGATCGCCGATCAGCGCGCCGCCGAGCAGTACGTGATTGACGAGTACAACAAGTTGAAAACTCGCCTGCGCGA

         2890       2900      2910      2920      2930      2940      2950      2960
AGCGGCACTGGCGTAATACCGTCCTTTCTACAGCCTCCTTTCGGAGGCTGTTTTTTTATCCATTCGAACTCTTTATACTG

         2970      2980      2990      3000      3010      3020      3030      3040
GTTACTTCCCG
```

## Beispiel 2

Vergleich der $\beta$-Galactosidase-Expression der Plasmide pBN208, pBN210 und pBN211.

Die wie in Beispiel 1 dargestellt, erhaltenen fdhF-lacZ-Fusionsplasmide wurden unter anaeroben und aeroben Wachstumsbedingungen auf Expression von $\beta$-Galactosidaseaktivität untersucht. Die $\beta$-Galactosidaseaktivität wurde nach J.H.Miller, Experiments in molecular genetics, Cold Spring Harbor Laboratory, Cold Spring Harbor, New York (1972) festgestellt und die spezifische Aktivität errechnet. Das Ergebnis ist in Fig. 1 dargestellt. In Fig. 1A sind die Ergebnisse der Expression unter anaeroben Bedingungen, im Teil B unter aeroben Wachstumsbedingungen gezeigt. Auf der Abszisse ist die Lokation der Deletion, das bedeutet die Position des Eco-Linkers vor dem Translationsstart (Position +1), angegeben. Eco-Linker bei -240 entspricht dem Plasmid pBN208, bei -184 dem Plasmid pBN210 und bei -143 dem Plasmid pBN211. Nitrat wurde in einer Menge von 10 mmol und Formiat mit einer Endkonzentration von 30 mmol zugegeben.

7

**Beispiel 3**

Herstellung des Plasmids pBN80, DSM 4073P

Das 1,11 Kb BamHI/PstI Fragment des Plasmids pBN208 wurde durch präparative Gelelektrophorese isoliert und anschließend in den, mit den Restriktionsendonukleasen PstI und BglII gespaltenen Vektor pGA46, DSM 4068P kloniert. In rekombinanten Plasmiden wird dadurch das β-Laktamasegen rekonstituiert. Das ligierte Plasmid pBN80, DSM 4073P, wurde, wie im Beispiel 1 für die Plasmide pBN2, pBN 208, pBN210, pBN211 und pMC 1403, beschrieben, in E. coli FM911 transformiert. Die Selektion nach Transformanten mit rekombinanten Plasmiden erfolgte daher nach Chloramphenicolresistenz (30 μg/ml) und Ampicillinresistenz (100 μg/ml). Die erhaltenen Klone wurden auf ihre Tetrazyklin-Resistenz unter aeroben und anaeroben Bedingungen untersucht. Auf Platten mit phosphatgepuffertem (pH 7) Vollmedium (mit 0,8% Glukose) sind die Klone unter aeroben Bedingungen Tetrazyklin-sensitiv. Unter anaeroben Bedingungen ist die Tetrazyklin-Resistenz exprimiert und wird mit Formiat (30 mM) induziert. Unter anaeroben Bedingungen in Gegenwart von 50 mM Nitrat sind die Klone dagegen Tetrazyklin-sensitiv.

**Beispiel 4**

Effekt einer ntrA-Mutation auf die Expression des fdhF-lacZ-Fusionsgens.

Zur Untersuchung der Auswirkungen der Mutation im ntrA-Gen wurde die β-Galactosidaseaktivität in zwei verschiedenen E. coli Stämmen, welche beide mit dem Plasmid pBN208, DSM 4069P, das das fdhF-lacZ-Fusionsgen enthält, transformiert waren, unter anaeroben und aeroben Bedingungen untersucht. Hierzu wurde der E. coli Stamm FM909, DSM 4279, der ntrA positiv ist, sowie der E. coli Stamm BN 950, DSM 4278, der ntrA negativ ist, verwendet. Die E. coli Stämme wurden in TGYEP-Medium, pH 6,5 (Begg et al., 1977, FEMS Mikrobiol. Let. 2:47-50), enthaltend 0,2 % (w/v) Glutamin und die angegebenen Mengen Formiat oder Nitrat, gezüchtet. Zusätzlich wurde der Stamm BN950, DSM 4278, mit drei weiteren Plasmiden, nämlich pBN17, DSM 4280P, pBN18 (s. Beispiel 6), und pBN61, DSM 4281P, transformiert. Die beiden Plasmide pBN17 und pBN18 enthalten das ntrA-Gen aus Klebsiella pneumoniae in unterschiedlicher Orientierung einligiert in einen Vektor. Das Plasmid pBN61 enthält das ntrA-Gen aus E. coli einligiert in einen Vektor. Es wird dadurch hergestellt, daß ein 2,7 kb großes Fragment aus E.coli-DNA in die BamHI-Schnittstelle des Vektors pACYC184 (J.Bacteriol. 134 (1978) 1141-1156) eingefügt wird.Auch für diese transformierten Stämme wurde die Expression der β-Galactosidase im angegebenen Wachstumsmedium der E. coli Stämme untersucht und in Tabelle 1 dargestellt.

**Beispiel 5**

Herstellung von pBN17

Das 1,96 kb ClaI-Fragment des Plasmids pMM17 (Sequenz: Nucl.Acids Res. 13 (1985) 7607-7620) wird in den Vektor pACYC184 (J.Bacteriol. 134 (1978) 1141-1156) in die ClaI-Schnittstelle kloniert. Hierbei wird der Vektor pBN17 (DSM 4280P) erhalten, der das ntrA-Gen enthält und bei Behandlung mit EcoRV in Fragmente der Länge 1,35 kb/750b und 3,83 kb gespalten wird.

**Beispiel 6**

Herstellung von pBN18

pBN18 enthält das ntrA-Gen in umgekehrter Orientierung wie pBN17 und wird durch Spaltung von pBN17 mit ClaI und anschließender Ligierung mit T₄-Ligase hergestellt.

Das Plasmid pBN18 ist dadurch charakterisiert, daß nach Behandlung mit EcoRV-Fragmente der Länge 1,35 kb/150b und 4,43 kb erhalten werden.

T a b e l l e   1

| Stamm | Genotyp | β-Galactosidase Aktivität (Einheiten) | | | | |
|---|---|---|---|---|---|---|
| | | Glucose (anaerob) | Glucose plus 30mM Formiat (anaerob) | Glucose plus 10mM Nitrat (anaerob) | Glucose (aerob) | Glucose plus 30mM Formiat (aerob) |
| FM909 (pBN208) | $\underline{ntrA}^+$ ($\underline{fdhF}$::$\underline{lacZ}$) | 340 | 1310 | 115 | 60 | 72 |
| BN950 (pBN208) | $\underline{ntrA}^-$ ($\underline{fdhF}$::$\underline{lacZ}$) | 235 | 380 | 130 | 52 | 60 |
| BN950 (pBN208, pBN17) | $\underline{ntrA}^-$ ($\underline{fdhF}$::$\underline{lacZ}$, °$\underline{ntrA}_k^+$) | 125 | 710 | 30 | n.d.* | n.d. |
| BN950 (pBN208, pBN18) | $\underline{ntrA}^-$ ($\underline{fdhF}$::$\underline{lacZ}$, °$\underline{ntrA}_k^+$) | 220 | 1075 | 85 | n.d. | n.d. |
| BN950 (pBN208, pBN61) | $\underline{ntrA}^-$ ($\underline{fdhF}$::$\underline{lacZ}$, °$\underline{ntrA}_e^+$) | 1083 | 3016 | 147 | n.d. | n.d. |

* n.d. = nicht bestimmt

° Der Index k bedeutet, daß dieses Gen aus Klebsiella pneumoniae stammt, der Index e bedeutet, daß dieses Gen aus Escherichia coli stammt.

**Patentansprüche**

1.  Rekombinante DNA, **dadurch gekennzeichnet,** daß sie die Consensus-Sequenz

```
       T   TTTC    T          A A   T
  AT   TCG       G   CACGTC A AC G
       G   AAAT   A          G C   G
```

und einen Promotor, der die DNA-Sequenz GGN$_{10}$GC aufweist, enthält.

**2.** Rekombinante DNA nach Anspruch 1, **dadurch gekennzeichnet,** daß sich die Consensus-Sequenz 15 bis 150 Basenpaare upstream vom Transkriptionsstart eines unter der Kontrolle des Promoters exprimierten Gens und der Promotor zwischen der Consensus-Sequenz und dem Transkriptionsstart befindet.

**3.** Rekombinante DNA nach Anspruch 2, **dadurch gekennzeichnet,** daß sich die Consensus-Sequenz 80 bis 130 bp vor dem Transkriptionsstart befindet.

**4.** Rekombinante DNA nach Anspruch 1 bis 3, **dadurch gekennzeichnet,** daß sie den Promotor einer für die Hydrogenase-Expression essentiellen Transkriptionseinheit oder den fdhF-Promotor enthält.

**5.** Rekombinante DNA nach Anspruch 1 bis 3, **dadurch gekennzeichnet,** daß sie essentielle Strukturelemente der ntrA-abhängigen Promotoren enthält.

**6.** Expressionsvektor, **dadurch gekennzeichnet,** daß er eine rekombinante DNA gemäß den Ansprüchen 1 bis 5 einligiert in einen geeigneten Vektor enthält.

**7.** Expressionsvektor nach Anspruch 6, **dadurch gekennzeichnet,** daß er zusätzlich einen Polylinker zum Einsetzen eines Fremdgens enthält.

**8.** Plasmid pBN80, DSM 4073P, **dadurch gekennzeichnet,** daß es den upstream-Bereich des fdhF-Gens von -240 bp bis zum Translationsstart einschließlich des fdhF-Promotors enthält.

**9.** Verfahren zur Herstellung einer rekombinanten DNA nach Anspruch 1 bis 5, **dadurch gekennzeichnet,** daß man eine die Consensus-Sequenz enthaltende DNA-Sequenz aus einer Genbank eines Mikroorganismus, der ein Gen enthält, das durch O$_2$ reprimierbar und unter anaeroben Bedingungen durch Formiat induzierbar ist, nach bekannten Methoden identifiziert, isoliert und mit einem geeigneten Promotor verbindet.

**10.** Verfahren nach Anspruch 9, **dadurch gekennzeichnet,** daß man die DNA-Sequenz aus der Genbank eines Mikroorganismus aus der Gruppe der Enterobacteriaceae isoliert.

**11.** Verfahren nach Anspruch 10, **dadurch gekennzeichnet,** daß man die DNA-Sequenz aus E. coli, DSM 2093 oder 2102, oder Salmonella typhimurium, DSM 554, isoliert.

**12.** Verfahren nach Anspruch 9 bis 11, **dadurch gekennzeichnet,** daß man die gesamte upstream-Region eines durch Formiat induzierbaren und durch O$_2$ reprimierbaren Gens, welche sowohl die Consensus-Sequenz als auch einen Promoter enthält, isoliert.

**13.** Verfahren nach Anspruch 12, **dadurch gekennzeichnet,** daß man die upstream-Region des fdhF-Gens isoliert.

**14.** Verfahren nach Anspruch 12, **dadurch gekennzeichnet,** daß man die upstream-Region des Promoters einer für die Hydrogenase-Expression essentiellen Transkriptionseinheit isoliert.

**15.** Verfahren nach Anspruch 9 bis 14 zur Herstellung eines Expressionsvektors nach Anspruch 6 oder 7, **dadurch gekennzeichnet,** daß man die isolierte rekombinante DNA-Sequenz, welche die Consensus-Sequenz und den Promoter enthält, gegebenenfalls mit einem Polylinker in einen geeigneten Vektor insertiert.

**16.** Verfahren nach Anspruch 13 zur Herstellung des Plasmids pBN80, DSM 4073P, nach Anspruch 8,

EP 0 285 152 B1

**dadurch gekennzeichnet,** daß man die upstream-Region des fdhF-Gens von -240 bp bis zum Translationsstart in den mit PstI und BglII geschnittenen Vektor pGA46, DSM 4068P, ligiert.

17. Verwendung einer rekombinanten DNA nach Anspruch 1 bis 5 und 8 oder eines Expressionsvektors nach Anspruch 6 und 7 zur induzierbaren und reprimierbaren Expression eines Fremdgens, **dadurch gekennzeichnet,** daß die Induktion unter anaeroben Bedingungen durch Formiat, die Repression durch $O_2$ bewirkt wird.

18. Verwendung nach Anspruch 17, **dadurch gekennzeichnet,** daß die Expression in E. coli oder Salmonella typhimurium, durchgeführt wird.

19. Verwendung nach Anspruch 17 oder 18, **dadurch gekennzeichnet,** daß die Expression in einem Wirtsstamm durchgeführt wird, der ntrA positiv ist.

20. Verwendung nach Anspruch 17 oder 18, **dadurch gekennzeichnet,** daß man die Expression in einem Wirtsstamm durchführt, der ntrA$^-$ ist und das ntrA-Gen in die rekombinante DNA oder den Expressionsvektor einligiert.

21. Verwendung nach Anspruch 17 oder 18, **dadurch gekennzeichnet,** daß man einen Wirtsstamm verwendet, der ntrA$^-$ ist und das ntrA- Gen auf einem zusätzlichen Vektor in die Wirtszellen einbringt.

22. Verwendung nach Anspruch 20 oder 21, **dadurch gekennzeichnet,** daß man ein dem der Wirtszelle entsprechendes ntrA-Gen verwendet.

**Claims**

1. Recombinant DNA, characterised in that it contains the consensus sequence:

```
 T    TTTC    T       A  A  T
AT   TCG       G   CACGTC  A  AC  G
 G    AAAT    A         G  C  G
```

and a promotor which has the DNA sequence $GGN_{10}GC$.

2. Recombinant DNA according to claim 1, characterised in that the consensus sequence is present 15 to 150 base pairs upstream from the transcription start of a gene expressed under the control of the promotor and the promotor is present between the consensus sequence and the transcription start.

3. Recombinant DNA according to claim 2, characterised in that the consensus sequence is present 80 to 130 bp before the transcription start.

4. Recombinant DNA according to claim 1 to 3, characterised in that it contains the promotor of a transcription unit essential for the hydrogenase expression or the fdhF promotor.

5. Recombinant DNA according to claim 1 to 3, characterised in that it contains essential structural elements of the ntrA-dependent promotors.

6. Expression vector, characterised in that it contains a recombinant DNA according to claims 1 to 5 ligated into a suitable vector.

7. Expression vector according to claim 6, characterised in that it additionally contains a polylinker for the introduction of a foreign gene.

8. Plasmid pBN80, DSM 4073P, characterised in that it contains the upstream region of the fdhF gene from -240 bp up to the translation start, including that of the fdhF promotor.

11

**9.** Process for the production of a recombinant DNA according to claims 1 to 5, characterised in that one identifies a DNA sequence containing the consensus sequence from a gene bank of a micro-organism, which contains a gene which is reprimable by $O_2$ and inducable by formate under anaerobic conditions, isolates and combines with a suitable promotor according to known methods.

**10.** Process according to claim 9, characterised in that one isolates the DNA sequence from the gene bank of a micro-organism of the Enterobacteriaceae group.

**11.** Process according to claim 10, characterised in that one isolates the DNA sequence from E. coli, DSM 2093 or 2102, or Salmonella typhimurium, DSM 554.

**12.** Process according to claim 9 to 11, characterised in that one isolates the whole upstream region of a gene inducable by formate and reprimable by $O_2$ which contains not only the consensus sequence but also a promotor.

**13.** Process according to claim 12, characterised in that one isolates the upstream region of the fdhF gene.

**14.** Process according to claim 12, characterised in that one isolates the upstream region of the promotor of a transcription unit essential for hydrogenase expression.

**15.** Process according to claim 9 to 14 for the production of an expression vector according to claim 6 or 7, characterised in that one inserts the isolated recombinant DNA sequence, which contains the consensus sequence and the promotor, into a suitable vector, optionally with a polylinker.

**16.** Process according to claim 13 for the production of the plasmid pBN80, DSM 4073P, according to claim 8, characterised in that one ligates the upstream region of the fdhF gene from -240 bp up to the translation start into the vector pGA46, DSM 4068P, cleaved with PstI and BglII.

**17.** Use of a recombinant DNA according to claim 1 to 5 and 8 or of an expression vector according to claims 6 and 7 for the inducable and reprimable expression of a foreign gene, characterised in that the induction is brought about under anaerobic conditions by formate, the repression by $O_2$.

**18.** Use according to claim 17, characterised in that the expression is carried out in E. coli or Salmonella typhimurium.

**19.** Use according to claim 17 or 18, characterised in that the expression is carried out in a host strain which is ntrA-positive.

**20.** Use according to claim 17 or 18, characterised in that one carries out the expression in a host strain which is ntrA$^-$ and ligates the ntrA gene into the recombinant DNA or the expression vector.

**21.** Use according to claim 17 or 18, characterised in that one uses a host strain which is ntrA$^-$ and introduces the ntrA gene into the host cell on an additional vector.

**22.** Use according to claim 20 or 21, characterised in that one uses an ntrA gene corresponding to the host cell.

**Revendications**

**1.** ADN recombiné, caractérisé en ce qu'il contient la séquence consensus

```
     T    TTTC    T         A  A    T
AT   TCG        G   CACGTC  A  AC  G
     G    AAAT    A           G  C   G
```

et un promoteur qui présente la séquence d'ADN GGN$_{10}$GC.

2. ADN recombiné selon la revendication 1, caractérisé en ce que la séquence consensus se trouve 15 à 150 paires de bases en amont du site d'initiation de la transcription d'un gène exprimé sous le contrôle du promoteur et le promoteur se trouve entre la séquence consensus et le site d'initiation de la transcription.

3. ADN recombiné selon la revendication 2, caractérisé en ce que la séquence consensus se trouve 80 à 130 paires de bases en amont du site d'initiation de la transcription.

4. ADN recombiné selon les revendications 1 à 3, caractérisé en ce qu'il contient le promoteur d'une unité de transcription essentielle pour l'expression de l'hydrogénase ou le promoteur fdhF.

5. ADN recombiné selon les revendications 1 à 3, caractérisé en ce qu'il contient les éléments structuraux essentiels des promoteurs ntrA-dépendants.

6. Vecteur d'expression caractérisé en ce qu'il contient un ADN recombiné selon les revendications 1 à 5, ligaturé dans un vecteur approprié.

7. Vecteur d'expression selon la revendication 6, caractérisé en ce qu'il contient en outre un lieur multisite pour l'introduction d'un gène étranger.

8. Plasmide pBN80, DSM 4073P, caractérisé en ce qu'il contient le domaine amont du gène fdhF de -240 pb jusqu'au site d'initiation de la traduction, y compris le promoteur fdhF.

9. Procédé de préparation d'un ADN recombiné selon les revendications 1 à 5, caractérisé en ce que, par des méthodes connues, on identifie et on isole, à partir d'une banque de gènes d'un micro-organisme qui contient un gène qui est réprimable par O$_2$ et inductible par le formiate dans des conditions anaérobies, une séquence d'ADN contenant la séquence consensus qu'on lie à un promoteur approprié.

10. Procédé selon la revendication 9, caractérisé en ce que l'on isole la séquence d'ADN à partir de la banque de gènes d'un micro-organisme du groupe des enterobacteriaceæ.

11. Procédé selon la revendication 10, caractérisé en ce que l'on isole la séquence d'ADN à partir de E. coli, DSM 2093 ou 2102, ou de Salmonella typhimurium, DSM 554.

12. Procédé selon les revendications 9 à 11, caractérisé en ce que l'on isole toute la région amont, contenant la séquence consensus et un promoteur, d'un gène inductible par le formiate et réprimable par O$_2$.

13. Procédé selon la revendication 12, caractérisé en ce que l'on isole la région amont du gène fdhF.

14. Procédé selon la revendication 12, caractérisé en ce que l'on isole la région amont du promoteur d'une unité de transcription essentielle pour l'expression de l'hydrogénase.

15. Procédé selon les revendications 9 à 14 pour la préparation d'un vecteur d'expression selon la revendication 6 ou 7, caractérisé en ce que l'on insère dans un vecteur approprié la séquence d'ADN recombiné isolée qui contient la séquence consensus et le promoteur, éventuellement avec un lieur multisite.

16. Procédé selon la revendication 13 pour la préparation du plasmide pBN80, DSM 4073P, selon la revendication 8, caractérisé en ce que l'on ligature la région amont du gène fdhF de -240 pb jusqu'au site d'initiation de la traduction dans le vecteur pGA46, DSM 4068P, coupé avec PstI et BgIII.

17. Utilisation d'un ADN recombiné selon les revendications 1 à 5 et 8 ou d'un vecteur d'expression selon les revendications 6 et 7 pour l'expression inductible et réprimable d'un gène étranger, caractérisé en ce que l'induction est réalisée dans des conditions anaérobies par le formiate et la répression est

réalisée par $O_2$.

**18.** Utilisation selon la revendication 17, caractérisée en ce que l'expression est réalisée dans E. coli ou Salmonella typhimurium.

**19.** Utilisation selon la revendication 17 ou 18, caractérisée en ce que l'expression est réalisée dans une souche hôte qui est ntrA-positive.

**20.** Utilisation selon la revendication 17 ou 18, caractérisée en ce qu'on réalise l'expression dans une souche hôte qui est ntrA⁻ et on ligature le gène ntrA dans l'ADN recombiné ou dans le vecteur d'expression.

**21.** Utilisation selon la revendication 17 ou 18, caractérisée en ce que l'on utilise une souche hôte qui est ntrA⁻ et l'on introduit dans les cellules hôtes le gène ntrA sur un vecteur supplémentaire.

**22.** Utilisation selon la revendication 20 ou 21, caractérisée en ce que l'on utilise un gène ntrA correspondant à celui de la cellule hôte.

β-Galactosidaseaktivität nach Expression
der Plasmide pBN208,pBN210,und pBN211.